# FASCICULE DE BREVET EUROPEEN

(11) **EP 4 312 823 B1**
(45) Date de publication et mention de la délivrance du brevet: **25.12.2024**
(21) Numéro de dépôt: 22718751.5
(22) Date de dépôt: 01.04.2022
(51) Int. Cl.: A61B 17/16, A61B 10/02, A61B 17/3205, A61B 17/34, A61B 17/00

(54) **DISPOSITIF DE PRÉLÈVEMENT DE MATIÈRE OSSEUSE**
VORRICHTUNG ZUR ENTNAHME VON KNOCHENMATERIALPROBEN
DEVICE FOR TAKING SAMPLES OF BONE MATTER

(30) Priorité: 02.04.2021 FR 2103443
(43) Date de publication de la demande: 07.02.2024
(73) Titulaire: L'etat Français Représenté Par Le Ministère De L'intérieur, 92130 Issy-les-Moulineaux (FR)
(72) Inventeur: MARTY, Julien, 95037 Cergy-Pontoise Cedex (FR)
(74) Mandataire: Yes My Patent
(86) Numéro de dépôt international: PCT/FR2022/050621
(87) Numéro de publication internationale: WO 2022/208036

(56) Documents cités:
- FR-A1- 2 625 429
- US-A- 5 556 399
- US-A- 5 632 747
- US-A1- 2002 058 945
- US-A1- 2008 234 715

## Description

La présente invention concerne un dispositif de prélèvement de matière osseuse destiné à être monté sur un outil rotatif de type outil électroportatif, perceuse orthopédique, ou encore moteur dentaire (ou tout autre type d'outil rotatif). Un tel dispositif de prélèvement est destiné notamment à des fins d'analyse génétique. En particulier, ce dispositif peut être utilisé dans le domaine de la sécurité, par exemple dans le cadre d'investigations criminalistiques ou forensiques aux fins d'analyses génétiques ou d'identification par empreintes génétiques. Le dispositif est également adapté à tout domaine dans lequel on cherche à prélever des échantillons de petite taille en minimisant la destruction de l'objet (aux fins de contre-analyse par exemple ou de conservation pour des musées).

L'analyse de l'ADN contenu dans les éléments osseux ou les dents est un processus long et complexe. La première étape indispensable de ce processus consiste à prélever une partie de la matrice osseuse. Cette étape est appelée l'échantillonnage. A l'issue de l'échantillonnage, la matrice osseuse prélevée est le plus souvent réduite à l'état de poudre par broyage mécanique. Il s'en suit une étape d'extraction d'ADN à partir de cette poudre. Cette étape d'extraction permet de lyser les cellules constituant la matrice osseuse afin d'en libérer l'ADN puis de purifier cet ADN des autres composants du lysat cellulaire. Dans le cadre de l'identification humaine par empreinte génétique, l'ADN ainsi purifié est amplifié par la technique de PCR afin de cibler et d'amplifier simultanément plusieurs régions sur l'ADN présentant un polymorphisme entre les individus. Les produits d'amplification ainsi obtenus sont enfin identifiés par des techniques de séquençage connues de l'homme du métier.

FR 2 625 429 A1 décrit un trocart mécanisé pour un prélèvement osseux. Le trocart comporte un trépan formé d'un corps tubulaire et un ensemble moteur l'entrainant en rotation.

US 2002/058945 A1 décrit un autre outil mécanisé pour un prélèvement osseux comportant un réservoir pour le prélèvement monté sur la poignée d'entrainement.

Actuellement, les techniques d'échantillonnage à partir d'éléments osseux aux fins d'identification par empreintes génétiques décrites dans la littérature ne permettent pas la mobilité, la standardisation, la simplicité, la rapidité et le traitement haut débit de manière sécurisée. En effet, la plupart des laboratoires utilisent soit à cette fin un marteau et une enclume, soit réalisent une découpe circulaire à l'aide de scies oscillantes ou rotatives communément utilisées en médecine légale lors des autopsies.

La première solution qui consiste à briser les os entre deux masses en acier, est encombrante et peut engendrer une projection non négligeable d'éclats, sans compter la présence dans le laboratoire d'une enclume difficilement décontaminable et transportable. Lors de cette opération, le manipulateur ne maîtrise pas la taille des débris obtenus et doit donc frapper à plusieurs reprises l'échantillon puis récupérer les fragments qu'il estime être de la bonne taille pour la suite des analyses.

Le laboratoire peut également choisir de s'équiper d'une scie oscillante ou rotative afin non plus de casser mais de découper les os en petits éléments. Cette technique nécessite toutefois une formation et une protection poussée de l'opérateur eu égard aux risques élevés de blessure pendant la manipulation de cet instrument. La découpe génère, de plus, une très grande quantité de poussière de matière osseuse qui nécessite également la protection de l'opérateur et de l'environnement. La taille des fragments osseux obtenus est encore bien souvent trop grande et requiert donc une seconde découpe plus précise s'avérant d'autant plus délicate notamment en raison de l'importante vitesse de rotation ou d'oscillation de la scie. Cette grande vitesse peut également engendrer une dégradation de l'ADN de l'échantillon en raison des échauffements induits. Aucune de ces solutions ne permet donc de travailler avec précision sur des échantillons centimétriques.

En alternative, il a été développé une méthode d'échantillonnage basée sur la découpe de matrices osseuses à l'aide de ciseaux de type ciseaux à bois. Les frottements du ciseau à bois à la surface de l'os par des mouvements rectilignes génèrent ainsi des copeaux. Cependant, en récoltant la surface de l'échantillon osseux, cette technique se limite à la zone la plus exposée aux agressions extérieures et par conséquent celle où l'ADN recherché a peu de chance d'être exploitable. La réalisation de cette technique nécessite en outre une longueur d'os suffisante pour une bonne prise en main ainsi qu'un savoir-faire certain pour recueillir de la matière osseuse et ne pas se blesser avec l'instrument. Elle s'avère également assez physique pour l'opérateur si un nombre important d'éléments osseux sont à traiter et peut générer en ce sens des troubles musculo-squelettiques (TMS). Les copeaux générés sont enfin très délicats à manipuler à l'aide d'une pince brucelles pour les disposer dans un microtube de 1,5 ou 2 ml couramment utilisés en biologie moléculaire en raison des phénomènes électrostatiques.

En outre, afin de rendre plus efficiente l'extraction et la purification de l'ADN, les protocoles les plus communément mis en oeuvre utilisent, à l'issue de l'échantillonnage, un broyeur de manière à réduire à l'état de poudre le fragment d'os prélevé.

Dans tous les cas, un tel broyage nécessite généralement de placer l'élément osseux à l'intérieur d'un bol en acier contenant une bille ce qui nécessite une décontamination complète entre chaque échantillon. Une fois la matière osseuse réduite à l'état de poudre, il est nécessaire d'en prélever entre 60 et 100 mg pour procéder à l'étape d'extraction de l'ADN. La pesée est effectuée à l'aide d'une balance de précision de laboratoire, l'opération consistant à disposer délicatement et successivement à l'aide d'une spatule la poudre d'os dans un microtube 1,5 ou 2 ml lui-même placé sur cette balance de manière à atteindre la masse de poudre ciblée. La succession d'étapes de transfert et de manipulation de l'échantillon est peu recommandée dans ce domaine étant donné la forte probabilité de contaminer l'échantillon, de perdre de la matière alors que l'ADN est déjà présent en faible quantité, voire de rompre la traçabilité.

Par conséquent, il est ici clairement mis en évidence que les méthodes d'échantillonnage employées à ce jour ne répondent pas aux critères de standardisation, de simplicité, de rapidité, de traitement haut débit et de sécurité.

Aussi, un premier but de la présente invention est de fournir un dispositif de prélèvement de matière osseuse permettant à un opérateur de pouvoir procéder facilement et avec précision à des échantillonnages centimétriques d'éléments osseux, et ce sans nécessiter d'effort physique trop important.

Un autre but de la présente invention est de fournir un dispositif de prélèvement de matière osseuse permettant la standardisation, la simplicité, la rapidité et le traitement haut débit de manière sécurisée.

Un autre but de la présente invention est de fournir un dispositif de prélèvement de matière osseuse transportable et permettant une décontamination rapide et efficace du dispositif entre deux prélèvements d'échantillons.

Une fois l'étape d'échantillonnage terminée, l'opérateur est donc la plupart du temps en présence de poudre d'os ou de copeaux dont il doit en extraire l'ADN. A cet effet, certains laboratoires utilisent encore une préparation chimique à base de phénolchloroforme et d'alcool isoamylique précédée d'une phase de décalcification (EDTA, Protéinase K). La durée d'un tel procédé peut facilement dépasser 24 heures, ce qui le rend incompatible avec un traitement rapide et haut débit, notamment dans le cadre d'identification de victimes.

Il existe en alternative plusieurs automates dédiés à l'extraction avec leurs réactifs associés. Il s'agit notamment de l'automate « Automate Express (ThermoFisher) » qui utilise la technologie des billes de silice magnétiques ou l'automate « Qiacube (QIAGEN) » qui utilise la technologie des colonnes de silice. Quel que soit l'automate choisi, l'étape de lyse cellulaire dure environ 2 heures 30 minutes puis la phase de purification au moins 1h avec des étapes de transferts de liquides en cours de protocole pouvant entraîner des contaminations croisées. De plus, ces instruments ne traitent qu'une dizaine d'échantillons à la fois et leurs poids de 55 à 70 kg permettent difficilement d'envisager leur transport en dehors du laboratoire pour procéder à des extractions d'ADN directement sur le terrain.

Aussi, un autre but de la présente invention est-il de fournir un ensemble permettant de réaliser à partir d'ossements à la fois l'étape d'échantillonnage et celle d'extraction d'ADN de manière standardisée, simple, rapide et à haut débit.

Un autre but de l'invention est de fournir un tel ensemble pour effectuer des prélèvements d'échantillons standards en vue d'en analyser l'ADN directement sur le site du prélèvement. Par « standardisation » on entend notamment le fait d'être toujours capable de produire un cylindre osseux identique, appelé carotte d'os, quel que soit le type d'os traité (long, plat, court, sésamoïde, etc.).

Ces buts, ainsi que d'autres qui apparaîtront par la suite, sont atteints par un dispositif de prélèvement de matière osseuse destiné à être monté sur un outil rotatif de type outil électroportatif, perceuse orthopédique, ou encore moteur dentaire, le dispositif comprenant :
- une partie de support disposée au niveau de l'extrémité proximale du dispositif, la partie de support étant munie de moyens d'accouplement à l'outil rotatif, pour l'entraînement en rotation du dispositif ;
- un trépan monté de manière amovible sur la partie de support, le trépan étant muni à son extrémité distale d'une tête coupante destinée à entrer en contact avec la matière osseuse ; le trépan et la partie de support étant creux et définissant ensemble un logement interne au sein du dispositif ; la tête coupante comportant à son extrémité libre une ouverture débouchant vers l'extérieur du dispositif, ladite ouverture étant en connexion fluidique avec ledit logement interne ; et
- un tube collecteur amovible, disposé au sein du logement interne du dispositif et ouvert à une de ses extrémités sensiblement en regard de l'ouverture de la tête coupante, le tube collecteur étant destiné à recueillir la matière osseuse découpée par le trépan.

La présence d'un tube collecteur amovible dans le dispositif de prélèvement de matière osseuse selon l'invention permet avantageusement d'utiliser ce tube pour des usages multiples au cours d'étapes successives d'échantillonnage et d'analyse. Ceci permet de minimiser le nombre de transferts de matière, et d'éviter ainsi de possibles contaminations polluantes d'échantillons, ainsi que de perdre la traçabilité des échantillons (en réduisant le nombre de manipulations de ces derniers). Le dispositif selon l'invention offre ainsi une mobilité, une standardisation, une simplicité, une rapidité et un traitement haut débit de manière sécurisée. En outre, le dispositif de prélèvement de matière osseuse est facilement transportable et permet une décontamination rapide et efficace du dispositif entre deux prélèvements d'échantillons.

De préférence, le tube collecteur est constitué d'un matériau métallique, typiquement de l'acier inoxydable ou du titane. Le choix du titane en particulier pour le tube collecteur permet une réutilisation du tube sur une très longue durée d'exploitation. Le choix du titane permet de décontaminer rapidement le tube collecteur entre deux échantillons sans risque d'oxydation.

Avantageusement, le tube collecteur est un consommable configuré pour être réutilisable après décontamination du tube. Ceci permet de réutiliser le même tube collecteur pour de nombreuses opérations de prélèvement, d'échantillonnage et d'analyse, et permet ainsi une réduction des coûts d'utilisation. En variante, le tube collecteur est un consommable à usage unique. Dans ce cas, il n'est pas constitué d'un matériau métallique, mais d'un matériau ayant un plus faible coût de production.

De préférence, le ou chaque tube collecteur comporte un marquage ou identifiant unique ; notamment, mais sans que cela ne soit limitatif, un code-barres, un QR code, une étiquette d'identification ou encore un numéro d'identification unique, typiquement un numéro d'identification apposé par gravure sur ou dans le tube collecteur. Ceci répond au besoin de traçabilité, qui entre dans le champ de la norme internationale ISO/CEI 17025 nécessaire notamment à l'accréditation COFRAC.

Selon un autre aspect, l'invention concerne également un kit pour le prélèvement de matière osseuse comprenant un dispositif de prélèvement de matière osseuse tel que décrit ci-dessus, un ou plusieurs tube(s) collecteur(s) destiné(s) à être monté(s) au sein du dispositif de prélèvement et/ou un ou plusieurs trépan(s) additionnels.

De préférence, le kit pour le prélèvement de matière osseuse comporte en outre un ou plusieurs capuchon(s) de fermeture de tube, le ou chaque capuchon de fermeture étant configuré pour fermer hermétiquement l'extrémité ouverte d'un tube collecteur lorsque le tube collecteur est hors du dispositif.

Selon un autre aspect, l'invention concerne également un ensemble comprenant un dispositif de prélèvement de matière osseuse tel que décrit ci-dessus ou un kit de prélèvement de matière osseuse tel que décrit ci-dessus ; ainsi qu'un dispositif de broyage de la matière osseuse prélevée, le dispositif de broyage de la matière osseuse étant configuré pour recevoir le tube collecteur du dispositif de prélèvement et pour broyer la matière osseuse contenue dans ledit tube collecteur. L'utilisation d'un tel dispositif de broyage de la matière osseuse permet de transformer la carotte d'os produite par dispositif de prélèvement, en poudre d'os. Une telle poudre d'os permet d'obtenir une surface de contact matière-réactifs plus grande, donnant de meilleurs profils génétiques pendant l'opération d'analyse génétique, et permettant ainsi de traiter des os plus anciens et/ou dégradés. Un tel ensemble selon l'invention est facilement transportable du fait de son faible encombrement, et de la portabilité des outils qu'il propose.

De préférence, le dispositif de broyage de la matière osseuse prélevée est un dispositif portatif, par exemple un pistolet de broyage.

Avantageusement, l'ensemble comporte en outre une bille disposée au sein du tube collecteur contenant la matière osseuse prélevée. Ceci permet de faciliter et de rendre plus homogène le broyage de la matière osseuse effectué par le dispositif de broyage. En particulier, l'ajout d'une telle bille directement dans le tube collecteur issu du dispositif de prélèvement, lorsqu'elle est combinée à la présence d'un capuchon de fermeture sur le tube collecteur, permet d'obtenir de la poudre d'os sans avoir à transférer de la matière entre les étapes d'échantillonnage et de broyage.

De préférence, le dispositif de broyage de matière osseuse est muni d'un adaptateur pour tubes, ledit adaptateur étant constitué d'un réceptacle amovible configuré pour recevoir au moins un tube collecteur, de préférence configuré pour recevoir deux tubes collecteurs. Ceci permet d'adapter très facilement au dispositif de broyage le tube collecteur issu du dispositif de prélèvement. Cette compatibilité offre un avantage substantiel en ce qu'elle permet d'éviter des manipulations et des transferts de matière générateurs de contamination. En outre, l'adapteur est configuré pour permettre un maintien efficace des tubes collecteurs métalliques dans le dispositif de broyage, pendant l'opération de broyage.

Selon un autre aspect, l'invention concerne également un laboratoire mobile formé d'un conteneur mobile transportable de type caisson roulant, pour la collecte, l'analyse et/ou l'identification de matériel biologique, le laboratoire mobile comprenant un dispositif de prélèvement tel que décrit ci-dessus ou un kit de prélèvement tel que décrit ci-dessus ou un ensemble tel que décrit ci-dessus, et étant configuré pour définir un espace de travail isolé pour un opérateur humain.

De préférence, le laboratoire mobile comprend en outre une colonne de perçage comportant un outil rotatif de type outil électroportatif, perceuse orthopédique, ou encore moteur dentaire ; et un étau destiné à maintenir des éléments osseux pendant un perçage. De tels éléments permettent de maintenir les éléments osseux pendant le perçage.

De préférence, le laboratoire mobile comprend en outre un moyen d'analyse génétique de type automate d'extraction d'ADN.

Selon un autre aspect, l'invention concerne également une utilisation d'un dispositif de prélèvement tel que décrit ci-dessus, dans laquelle si le prélèvement est effectué avec le dispositif directement sur un être humain, ledit être humain n'est pas vivant. Une telle utilisation est par exemple particulièrement adaptée pour des médecins légistes, afin de leur éviter une dissection importante et longue aux fins d'ablation du fémur lors des autopsies.

Il est précisé que :
- par échantillonnage standardisé, on entend désigner selon la présente invention un prélèvement de matière aux caractéristiques définies et déterminées comme étant une norme répétable.
- par matrice osseuse, on entend un échantillon biologique, pouvant être issu de la totalité ou d'une partie du tissu osseux ou dentaire d'un être vivant, décédé au moment de la collecte, par exemple un être humain.
- par analyse génétique rapide et haut débit, on entend l'obtention d'un profil génétique le plus généralement par génotypage type STR (de l'anglais Short Tandem Repeat). En cas d'identification de victimes de masse, le génotypage de STR sera préférentiellement choisi et le protocole devra permettre d'obtenir en quelques heures un grand nombre de profils génétiques.

La description qui va suivre et qui ne présente aucun caractère limitatif, doit être lue en regard des figures annexées, parmi lesquelles :
- la figure 1 est une vue en perspective d'un exemple de dispositif de prélèvement de matière osseuse selon la présente invention, le dispositif comprenant un tube collecteur amovible ;
- la figure 2 est une vue de côté du dispositif de prélèvement de la figure 1 ;
- la figure 3 est une vue en coupe longitudinale, prise selon le plan de coupe III-III, du dispositif de prélèvement de la figure 2 ;
- la figure 4 est une vue en perspective d'un exemple de réalisation d'un adaptateur pour tubes, un tel adaptateur étant configuré pour recevoir le tube collecteur de la figure 1 et étant destiné à équiper un dispositif de broyage de matière osseuse ; et
- la figure 5 est une vue en perspective d'un exemple de laboratoire mobile selon l'invention, sous forme de conteneur mobile transportable, le laboratoire mobile comprenant le dispositif de prélèvement de matière osseuse de la figure 1.

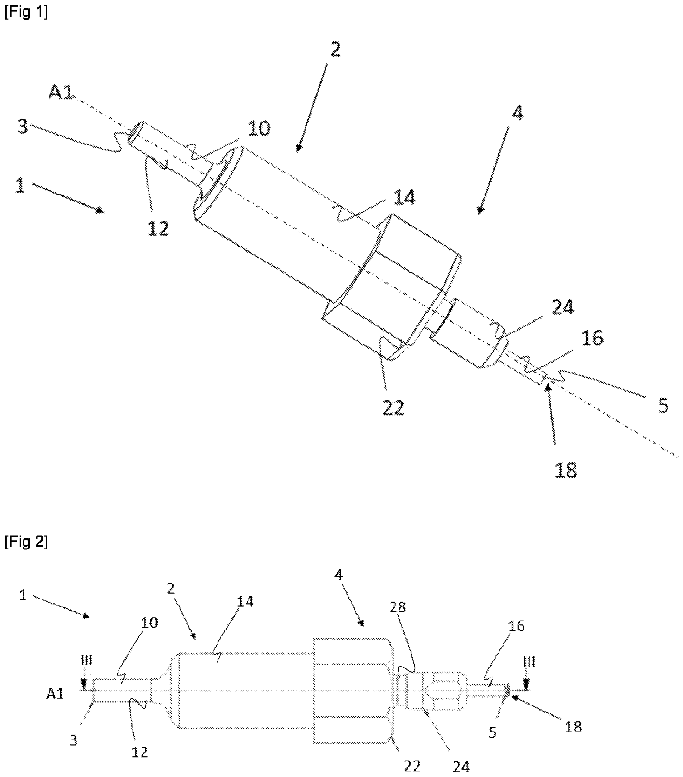

Les figures 1 à 3 représentent un dispositif 1 de prélèvement de matière osseuse selon l'invention. Le dispositif 1 est destiné à être monté sur un outil rotatif (non représenté) de type outil électroportatif, perceuse orthopédique, ou encore moteur dentaire, permettant l'entraînement en rotation du dispositif 1. Le dispositif 1 s'étend longitudinalement le long d'un axe A1 (qui est aussi son axe de rotation), et présente une extrémité proximale 3, destinée à être reliée à l'outil rotatif, et une extrémité distale 5 destinée à découper la matière osseuse. Le dispositif 1 fait avantageusement partie d'un ensemble (non représenté) comprenant, outre le dispositif de prélèvement 1, un dispositif de broyage de la matière osseuse prélevée (un tel dispositif de broyage n'étant pas représenté sur les figures). Sans que cela ne soit limitatif, le dispositif de broyage est typiquement un dispositif portatif de type pistolet de broyage. Le pistolet de broyage est typiquement en forme de « T ». Ce pistolet de broyage peut être, par exemple, un pistolet de broyage portatif tel que l'instrument Super FastPrep-2 de la marque MP BIOMEDICALSO. En variante ou de manière complémentaire, le dispositif 1 peut également faire partie d'un laboratoire mobile 40, comme cela sera décrit par la suite.

Le dispositif de prélèvement 1 comporte une partie de support 2 et un trépan 4 monté de manière amovible sur la partie de support 2. Le trépan 4 et la partie de support 2 sont creux et définissent ensemble un logement interne 6 au sein du dispositif 1. Le dispositif de prélèvement 1 comporte en outre un tube collecteur amovible 8, disposé au sein du logement interne 6. Le tube collecteur 8 est destiné à recueillir la matière osseuse découpée par le trépan 4.

La partie de support 2 est disposée au niveau de l'extrémité proximale 3 du dispositif 1 et forme une pièce rotative qui est reliée à l'outil rotatif. Pour ce faire, un mandrin (par exemple de type « quick connect ») peut relier la partie de support 2 à l'outil rotatif. Tout type de mandrin peut être utilisé, par exemple un mandrin de type mandrin de perceuse universel et automatique. La partie de support 2 forme une pièce généralement tronconique et tubulaire, et comporte des moyens 10 d'accouplement à l'outil rotatif, notamment au mandrin de connexion à ce dernier. Les moyens d'accouplement 10 sont par exemple constitués d'une portion proximale 12 de la partie 2, plus fine en diamètre qu'une portion distale 14 de cette dernière, et destinée à être insérée et maintenue par serrage dans le mandrin. La partie de support 2 est par exemple constituée d'acier inoxydable.

Le trépan 4 est par exemple monté sur la partie de support 2 au moyen d'un outil de serrage de type pince. Le trépan 4 est muni à son extrémité distale 5 d'une tête coupante 16 destinée à entrer en contact avec la matière osseuse. La tête coupante 16 comporte à son extrémité libre une ouverture 18 qui débouche à l'extérieur du dispositif 1. L'ouverture 18 est à l'extrémité d'un canal interne 20 qui s'étend longitudinalement à l'intérieur du trépan 4, le long de l'axe A1. Le canal interne 20 est en connexion fluidique avec le logement interne 6. La tête coupante 16 comporte une couronne qui pénètre en rotation dans la matière osseuse et en découpe des éléments qui sont évacués dans la canal interne 20 via l'ouverture 18.

Dans l'exemple de réalisation particulier illustré sur les figures 1 à 3, le trépan 4 comporte, outre la tête coupante 16, une pièce d'adaptation 22 destinée à être montée (notamment par vissage) sur la partie de support 2, ainsi qu'un écrou 24 reliant la pièce d'adaptation 22 à la tête coupante 16. La pièce d'adaptation 22 et l'écrou 24 sont creux et définissent ensemble le canal interne 20. La pièce d'adaptation 22 présente une forme sensiblement tronconique, qui définit une symétrie de révolution. L'écrou 24, qui est par exemple un écrou de mandrin, est typiquement fixé sur la pièce d'adaptation 22 au moyen d'une pince creuse interne élastiquement déformable 26. La pince creuse interne 26 est solidaire de l'écrou 24 et insérée dans une ouverture 27 débouchant depuis un manchon 28 s'étendant à une extrémité de la pièce d'adaptation 22. L'effet élastique des branches de la pince 26 permet ainsi de retenir l'écrou 24 sur la pièce 22 et d'empêcher tout déplacement longitudinal de ce dernier lors de l'utilisation du dispositif 1. La paroi externe de l'écrou 24 s'étend alors autour du manchon 28. La tête coupante 16 est insérée dans l'écrou de mandrin 24 et maintenue par serrage dans ce dernier. Via cet agencement entre la partie de support 2 et le trépan 4, le trépan 4 est ainsi configuré pour être entrainé en rotation par la partie de support 2 lorsque cette dernière est reliée à l'outil rotatif, la rotation s'effectuant autour de l'axe de rotation A1. Le trépan 4 est par exemple constitué d'acier inoxydable. Typiquement, la pièce d'adaptation 22 est par exemple en inox de qualité SAE 316L, tandis que la tête coupante 16 peut être en inox de type X30Cr13. Les qualités recherchées sont la longévité de l'aiguisage pour diminuer les échauffements et la non altération par oxydation suite à la décontamination.

Le tube collecteur 8 est fermé à une de ses extrémités 29A, et ouvert à son autre extrémité 29B. L'extrémité ouverte 29B du tube 8 est disposée sensiblement en regard du canal interne 20, et donc de l'ouverture 18 de la tête coupante 16. Le tube collecteur 8, disposé dans le logement interne 6, est logé en partie dans un évidement 31 ménagé à l'intérieur de la partie de support 2. Une rondelle d'appui 25 peut être insérée autour de l'extrémité ouverte 29B du tube 8, entre le tube 8 et une douille interne 33 faisant saillie depuis la paroi interne de la pièce 22, pour faciliter l'assemblage de l'ensemble. Le tube collecteur 8 est de préférence constitué d'un matériau métallique, typiquement de l'acier inoxydable ou du titane. Selon un mode de réalisation particulier de l'invention, le tube collecteur 8 est un consommable configuré pour être réutilisable après décontamination du tube. Selon un autre mode de réalisation particulier, le tube collecteur 8 peut contenir une bille (non représentée sur les figures), par exemple une bille en métal, et notamment en acier inoxydable.

Le tube collecteur 8 comporte de préférence un marquage ou identifiant unique ; notamment, mais sans que cela ne soit limitatif, un code-barres, un QR code, une étiquette d'identification ou encore un numéro d'identification unique, typiquement un numéro d'identification apposé par gravure sur ou dans le tube collecteur 8.

Le dispositif de prélèvement 1 se veut léger, robuste, d'utilisation sécurisée pour son opérateur et facilement décontaminable notamment par stérilisation pour éviter tout risque de contamination entre les échantillons. Le dispositif de prélèvement 1 permet de découper de la matière osseuse sous forme d'une carotte qui sera recueillie dans le tube collecteur 8. Les carottes osseuses obtenues sont alors toutes de longueur et de diamètre standardisés. Elles peuvent ainsi être facilement manipulées, transportées ou stockées dans des tubes collecteurs 8. Le carottage est réalisé préférentiellement à faible vitesse de rotation en fonction des caractéristiques de l'outil rotatif choisi.

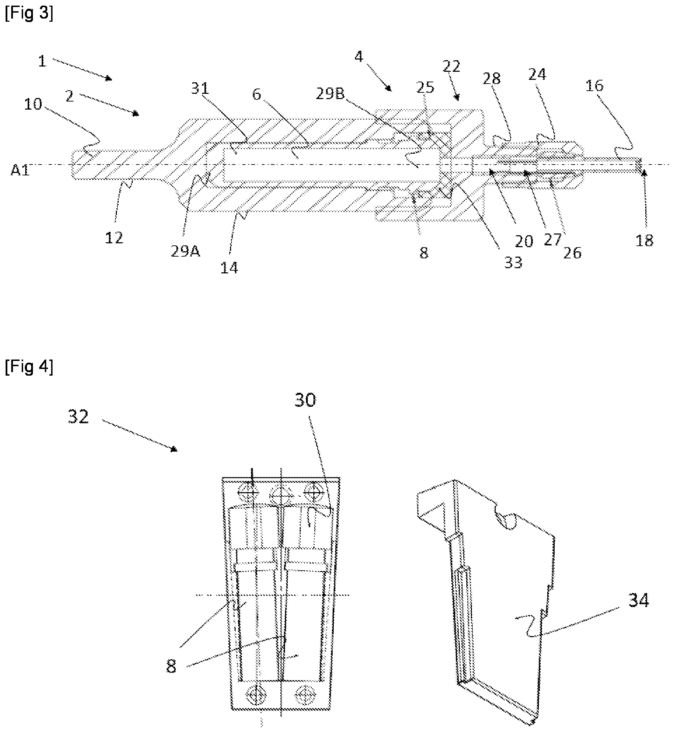

Une fois enlevé du dispositif de prélèvement 1 (par désolidarisation manuelle du trépan 4 vis-à-vis de la partie de support 2), le tube collecteur 8 contenant la matière osseuse prélevée peut être refermé par un capuchon 30 de fermeture du tube (visible sur la figure 4). Le capuchon de fermeture 30 est configuré pour fermer hermétiquement l'extrémité ouverte 29B du tube collecteur 8. Le tube collecteur 8 peut alors être placé, par exemple, dans le dispositif de broyage de matière osseuse. Pour ce faire, le dispositif de broyage peut être muni d'un adaptateur 32 pour tubes, un tel adaptateur 32 étant représenté sur la figure 4. L'adaptateur 32 est constitué d'un réceptacle amovible configuré pour recevoir le tube collecteur 8. De préférence, et comme illustré sur la figure 4, le réceptacle 32 est configuré pour recevoir deux tubes collecteurs 8 distincts. Le réceptacle 32 est préférentiellement fermé par un couvercle 34 et est destiné à être fixé au sein du dispositif de broyage, par exemple par vissage. Le couvercle 34 est glissé dans une rainure 36 prévue sur le réceptacle, puis est retenu et serré au moyen d'une vis (non représentée) qui vient se fixer entre les deux capuchons 30 de fermeture de tube.

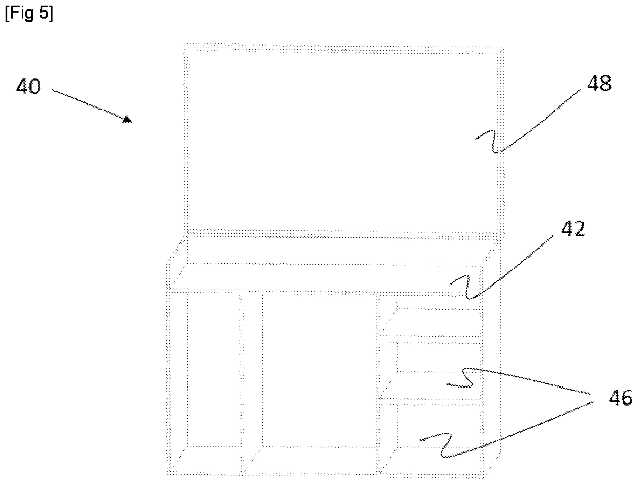

Sur la figure 5 est représenté un laboratoire mobile 40 pour la collecte, l'analyse et/ou l'identification de matériel biologique, comprenant le dispositif de prélèvement 1 selon l'invention. Le laboratoire mobile 40 peut également comporter le dispositif de broyage portatif, muni de son adaptateur 32.

Le laboratoire mobile 40 est formé d'un conteneur mobile transportable de type caisson roulant, et est configuré de manière à définir un espace de travail isolé pour un opérateur humain. Pour ce faire, le caisson roulant 40 comporte par exemple une paillasse 42 montée sur quatre pieds roulants (non représentés sur les figures), munie de rangements 46 et surmontée d'un couvercle 48. Une table d'extension (non représenté), montée sur pieds roulants, peut également être prévue dans le caisson roulant 40.

Outre le dispositif de prélèvement 1 et le dispositif de broyage, le laboratoire mobile 40 peut aussi comprend un moyen pour l'analyse génétique de la matière osseuse prélevée issue du dispositif de broyage. Ce moyen d'analyse génétique (non représenté sur les figures pour des raisons de clarté) peut être, par exemple, un automate portatif permettant l'extraction d'ADN tel que l'instrument PDQex 2400 de la marque Zygem^{®} permettant de traiter 24 échantillons en 20 minutes. Les réactifs utilisés pour la phase d'extraction sont également optimisés pour traiter des matrices osseuses.

Le laboratoire mobile 40 peut également comporter une colonne de perçage comportant un outil rotatif de type outil électroportatif, perceuse orthopédique, ou encore moteur dentaire ; ainsi qu'un étau destiné à maintenir des éléments osseux pendant un perçage. La colonne de perçage, l'outil électroportatif et l'étau ne sont pas représentés sur la figure 5. De tels éléments permettent de maintenir les éléments osseux pendant le perçage.

Une des utilisations de la présente solution est l'identification de victimes de masse suite par exemple à une catastrophe aérienne se traduisant par plusieurs centaines ou milliers de restes humains fragmentés et dans un état très dégradé. Selon le protocole d'analyse ADN utilisé conventionnellement aux fins d'identification des victimes (qui peut être typiquement un protocole ICMP - de l'anglais International Commission on Missing Persons), il faut acheminer chacun de ces éléments osseux au laboratoire d'analyse dans des conditions réfrigérées, puis échantillonner et analyser chacun de ces fragments. Cette opération nécessite avec les solutions actuelles une logistique lourde et complexe compte tenu du volume de stockage qu'impose ce genre de catastrophe.

Au contraire, la mise en oeuvre de la présente invention permet dans un tel cas de réaliser des carottages de matrices osseuses sur chacun des éléments osseux au plus près du site de la catastrophe. Les études préliminaires effectuées sur différents types d'os humain tendent à démontrer que la quantité de matière nécessaire à l'obtention d'un profil ADN correspond à 3 ou 5 carottes d'os. Les carottes issues de chaque élément osseux échantillonné peuvent ainsi être rassemblées dans un tube collecteur par exemple de 2ml présentant un identifiant code-barres unique. A l'issue de la phase d'échantillonnage, les échantillons peuvent être soit directement analysés sur site au sein d'un laboratoire mobile ADN, soit acheminés à un laboratoire conventionnel. La logistique s'en retrouve alors grandement simplifiée : selon la mise en oeuvre de cette solution, 96 échantillons peuvent ainsi être stockés dans une cryo-boîte (par exemple une cryo-boîte aux dimensions de (Lxlxh) 25x12x5 cm).

Une autre utilisation de la présente solution serait de procéder à l'identification par l'ADN d'éléments osseux rassemblés dans des charniers. Là encore, la simplicité, la rapidité et la standardisation de la méthode d'échantillonnage permettent de réaliser directement les prélèvements et l'analyse sur place ou à défaut d'acheminer uniquement les échantillons au laboratoire tout en stockant les ossements sur le site du charnier.

La présente invention a été décrite en référence à un dispositif 1 de prélèvement de matière osseuse comprenant un seul trépan 4 et un seul tube collecteur 8. L'invention concerne également un kit pour le prélèvement de matière osseuse comprenant, outre le dispositif de prélèvement 1, un ou plusieurs tube(s) collecteur(s) 8 et/ou un ou plusieurs trépan(s) additionnels 4 (de différentes tailles et/ou diamètres). Le kit peut également comporter un ou plusieurs capuchon(s) 30 de fermeture de tube 8.

## Revendications

1. - Dispositif (1) de prélèvement de matière osseuse destiné à être monté sur un outil rotatif de type outil électroportatif, perceuse orthopédique, ou encore moteur dentaire, le dispositif (1) comprenant :
- une partie de support (2) disposée au niveau de l'extrémité proximale (3) du dispositif (1), la partie de support (2) étant munie de moyens (10) d'accouplement à l'outil rotatif, pour l'entraînement en rotation du dispositif (1) ; et
- un trépan (4) monté de manière amovible sur la partie de support (2), le trépan (4) étant muni à son extrémité distale (5) d'une tête coupante (16) destinée à entrer en contact avec la matière osseuse ; le trépan (4) et la partie de support (2) étant creux et définissant ensemble un logement interne (6) au sein du dispositif (1); la tête coupante (16) comportant à son extrémité libre une ouverture (18) débouchant vers l'extérieur du dispositif (1), ladite ouverture (18) étant en connexion fluidique avec ledit logement interne (6); **caractérisé en ce que** le dispositif comprend:
- un tube collecteur amovible (8), disposé au sein du logement interne (6) du dispositif (1) et ouvert à une de ses extrémités (29B) sensiblement en regard de l'ouverture (18) de la tête coupante (16), le tube collecteur (8) étant destiné à recueillir la matière osseuse découpée par le trépan (4).

2. - Dispositif de prélèvement (1) selon la revendication 1, **caractérisé en ce que** le tube collecteur (8) est constitué d'un matériau métallique, typiquement de l'acier inoxydable ou du titane.

3. - Dispositif de prélèvement (1) selon la revendication 1 ou 2, **caractérisé en ce que** le tube collecteur (8) est un consommable configuré pour être réutilisable après décontamination du tube (8).

4. - Kit pour le prélèvement de matière osseuse comprenant un dispositif de prélèvement (1) de matière osseuse, un ou plusieurs tube(s) collecteur(s) (8) destiné(s) à être monté(s) au sein du dispositif de prélèvement (1) et/ou un ou plusieurs trépan(s) (4) additionnels, **caractérisé en ce que** le dispositif (1) de prélèvement de matière osseuse est conforme à l'une quelconque des revendications précédentes.

5. - Kit de prélèvement selon la revendication 4, **caractérisé en ce qu'**il comporte en outre un ou plusieurs capuchon(s) (30) de fermeture de tube (8), le ou chaque capuchon de fermeture (30) étant configuré pour fermer hermétiquement l'extrémité ouverte (29B) d'un tube collecteur (8) lorsque le tube collecteur (8) est hors du dispositif (1).

6. - Ensemble comprenant un dispositif (1) de prélèvement de matière osseuse selon l'une quelconque des revendications 1 à 3 ou un kit de prélèvement de matière osseuse selon la revendication 4 ou 5 ; ainsi qu'un dispositif de broyage de la matière osseuse prélevée, le dispositif de broyage de la matière osseuse étant configuré pour recevoir le tube collecteur (8) du dispositif de prélèvement (1) et pour broyer la matière osseuse contenue dans ledit tube collecteur (8).

7. - Ensemble selon la revendication 6, **caractérisé en ce que** le dispositif de broyage de la matière osseuse prélevée est un dispositif portatif, par exemple un pistolet de broyage.

8. - Ensemble selon la revendication 6 ou 7, **caractérisé en ce qu'**il comporte en outre une bille disposée au sein du tube collecteur (8) contenant la matière osseuse prélevée.

9. - Ensemble selon l'une quelconque des revendications 6 à 8, **caractérisé en ce que** le dispositif de broyage de matière osseuse est muni d'un adaptateur (32) pour tubes (8), ledit adaptateur (32) étant constitué d'un réceptacle amovible configuré pour recevoir au moins un tube collecteur (8), de préférence configuré pour recevoir deux tubes collecteurs (8).

10. - Laboratoire mobile (40) formé d'un conteneur mobile transportable de type caisson roulant, pour la collecte, l'analyse et/ou l'identification de matériel biologique, le laboratoire mobile (40) comprenant un dispositif de prélèvement (1) selon l'une des revendications 1 à 3 ou un kit de prélèvement selon l'une des revendications 4 à 5 ou un ensemble selon l'une des revendications 6 à 9, et étant configuré pour définir un espace de travail isolé pour un opérateur humain.

11. - Laboratoire mobile (40) selon la revendication 10, **caractérisé en ce qu'**il comprend en outre une colonne de perçage comportant un outil rotatif de type outil électroportatif, perceuse orthopédique, ou encore moteur dentaire ; et un étau destiné à maintenir des éléments osseux pendant un perçage.

12. - Laboratoire mobile (40) selon la revendication 10 ou 11, **caractérisé en ce qu'**il comprend en outre un moyen d'analyse génétique de type automate d'extraction d'ADN.

13. - Utilisation d'un dispositif (1) selon l'une quelconque des revendications 1 à 3 pour le prélèvement de matière osseuse, **caractérisée en ce que** si le prélèvement est effectué avec le dispositif (1) directement sur un être humain, ledit être humain n'est pas vivant.

## Patentansprüche

1. - Vorrichtung (1) für die Entnahme von Knochenmaterie, die dafür bestimmt ist, an einem rotierenden Werkzeug einer Elektrowerkzeugart, einer orthopädischen Bohrmaschine oder sogar einem Dentalmotor, montiert zu werden, wobei die Vorrichtung (1) umfasst:
- ein Trägerteil (2), das an dem proximalen Ende (3) der Vorrichtung (1) angeordnet ist, wobei das Trägerteil (2) mit Mitteln (10) für eine Ankopplung an das rotierende Werkzeug für einen Drehantrieb der Vorrichtung (1) versehen ist; und
- einen Trepan (4), der an dem Trägerteil (2) abnehmbar montiert ist, wobei der Trepan (4) an seinem distalen Ende (5) mit einem Schneidkopf (16) versehen ist, der dafür bestimmt ist, mit der Knochenmaterie in Kontakt zu kommen; der Trepan (4) und das Trägerteil (2) hohl sind und zusammen ein Innengehäuse (6) innerhalb der Vorrichtung (1) definieren; wobei der Schneidkopf (16) an seinem freien Ende eine Öffnung (18) aufweist, die sich zu der Außenseite der Vorrichtung (1) hin öffnet, wobei die Öffnung (18) in Fluidverbindung mit dem Innengehäuse (6) steht;
**dadurch gekennzeichnet, dass** die Vorrichtung umfasst:
- ein abnehmbares Sammelrohr (8), das innerhalb des Innengehäuses (6) der Vorrichtung (1) angeordnet ist und an einem seiner Enden (29B) im Wesentlichen gegenüber der Öffnung (18) des Schneidkopfs (16) offen ist, wobei das Sammelrohr (8) dafür bestimmt ist, die durch den Trepan (4) abgeschnittene Knochenmaterie aufzufangen.

2. - Entnahmevorrichtung (1) nach Anspruch 1,
**dadurch gekennzeichnet, dass** das Sammelrohr (8) aus einem metallischen Material, typischerweise Edelstahl oder Titan, besteht.

3. - Entnahmevorrichtung (1) nach Anspruch 1 oder 2,
**dadurch gekennzeichnet, dass** das Sammelrohr (8) ein Verbrauchsmaterial ist, das konfiguriert ist, um nach einer Dekontamination des Rohrs (8) wiederverwendbar zu sein.

4. - Kit für die Entnahme von Knochenmaterie, umfassend eine Vorrichtung (1) für die Entnahme von Knochenmaterie, ein oder mehrere Sammelrohr(e) (8), die dafür bestimmt sind, innerhalb der Entnahmevorrichtung (1) montiert zu werden, und/oder einen oder mehrere zusätzliche Trepan(e) (4),
**dadurch gekennzeichnet, dass** die Vorrichtung (1) für die Entnahme von Knochenmaterie einem der vorstehenden Ansprüche entspricht.

5. - Entnahmekit nach Anspruch 4, **dadurch gekennzeichnet, dass** es ferner eine oder mehrere Kappe(n) (30) für einen Verschluss des Rohrs (8) aufweist, wobei die oder jede Verschlusskappe (30) konfiguriert ist, um das offene Ende (29B) eines Sammelrohrs (8) hermetisch zu verschließen, wenn das Sammelrohr (8) sich außerhalb der Vorrichtung (1) befindet.

6. - Anordnung, umfassend eine Vorrichtung (1) für die Entnahme von Knochenmaterie nach einem der Ansprüche 1 bis 3 oder Kit für die Entnahme von Knochenmaterie nach Anspruch 4 oder 5; sowie eine Vorrichtung zum Zerkleinern der entnommenen Knochenmaterie, wobei die Vorrichtung zum Zerkleinern der Knochenmaterie konfiguriert ist, um das Sammelrohr (8) der Entnahmevorrichtung (1) aufzunehmen und um die in dem Sammelrohr (8) enthaltene Knochenmaterie zu zerkleinern.

7. - Anordnung nach Anspruch 6, **dadurch gekennzeichnet, dass** die Vorrichtung zum Zerkleinern der entnommenen Knochenmaterie eine tragbare Vorrichtung, beispielsweise eine Zerkleinerungspistole, ist.

8. - Anordnung nach Anspruch 6 oder 7, **dadurch gekennzeichnet, dass** sie ferner eine Kugel, die innerhalb des Sammelrohrs (8) angeordnet ist, das die entnommene Knochenmaterie enthält, aufweist.

9. - Anordnung nach einem der Ansprüche 6 bis 8,
**dadurch gekennzeichnet, dass** die Vorrichtung für die Zerkleinerung der Knochenmaterie mit einem Adapter (32) für die Rohre (8) versehen ist, wobei der Adapter (32) aus einem abnehmbaren Behälter besteht, der für die Aufnahme mindestens eines Sammelrohrs (8) konfiguriert ist, vorzugsweise für die Aufnahme von zwei Sammelrohren (8) konfiguriert ist.

10. - Mobiles Labor (40), das aus einem transportablen mobilen Container einer Rollkastenart ausgebildet ist, für die Sammlung, die Analyse und/oder die Identifizierung von biologischem Material, wobei das mobile Labor (40) eine Entnahmevorrichtung (1) nach einem der Ansprüche 1 bis 3 oder ein Entnahmekit nach einem der Ansprüche 4 bis 5 oder eine Anordnung nach einem der Ansprüche 6 bis 9 umfasst und konfiguriert ist, um einen isolierten Arbeitsbereich für einen menschlichen Bediener zu definieren.

11. - Mobiles Labor (40) nach Anspruch 10, **dadurch gekennzeichnet, dass** es ferner eine Bohrsäule umfasst, aufweisend ein rotierendes Werkzeug, einer Elektrowerkzeugart, einen orthopädischen Bohrer oder sogar einen Dentalmotor, und einen Schraubstock, der dafür bestimmt ist, Knochenelemente während eines Bohrens zu halten.

12. - Mobiles Labor (40) nach Anspruch 10 oder 11,
**dadurch gekennzeichnet, dass** es ferner ein Mittel für die genetische Analyse einer automatischen DNA-Extraktionsart umfasst.

13. - Verwendung einer Vorrichtung (1) nach einem der Ansprüche 1 bis 3 für die Entnahme von Knochenmaterie, **dadurch gekennzeichnet, dass,** falls die Entnahme mit der Vorrichtung (1) direkt an einem menschlichen Wesen vorgenommen wird, das menschliche Wesen nicht lebend ist.

## Claims

1. Device (1) for taking samples of bone matter, which device is intended to be mounted on a rotary tool such as a portable power tool, an orthopedic drill, or even a dental motor, the device (1) comprising:
- a support part (2) positioned at the proximal end (3) of the device (1), the support part (2) being equipped with means (10) for coupling to the rotary tool, so as to rotationally drive the device (1); and
- a trephine (4) mounted removably on the support part (2), the trephine (4) being equipped at its distal end (5) with a cutting head (16) intended to come into contact with the bone matter; the trephine (4) and the support part (2) being hollow and together defining an internal housing (6) within the device (1); the cutting head (16) comprising at its free end an opening (18) opening to the outside of the device (1), the opening (18) being in fluidic connection with the internal housing (6); **characterized in that** the device comprises:
- a removable collecting tube (8), positioned within the internal housing (6) of the device (1) and open at one of its ends (29B) substantially facing the opening (18) of the cutting head (16), the collecting tube (8) being intended to collect the bone matter cut by the trephine (4).

2. Sample-taking device (1) according to claim 1, **characterized in that** the collecting tube (8) consists of a metal material, typically stainless steel or titanium.

3. Sample-taking device (1) according to either claim 1 or claim 2, **characterized in that** the collecting tube (8) is a consumable configured to be reusable after decontamination of the tube (8).

4. Kit for taking samples of bone matter, comprising a device (1) for taking samples of bone matter, one or more collecting tube(s) (8) intended to be mounted within the sample-taking device (1) and/or one or more additional trephine(s) (4), **characterized in that** the device (1) for taking samples of bone matter is in accordance with any of the preceding claims.

5. Sample-taking kit according to claim 4, **characterized in that** it further comprises one or more cap(s) (30) for closing the tube (8), the or each closure cap (30) being configured to hermetically close the open end (29B) of a collecting tube (8) when the collecting tube (8) is outside the device (1).

6. Assembly comprising a device (1) for taking samples of bone matter according to any of claims 1 to 3 or a kit for taking samples of bone matter according to either claim 4 or claim 5; as well as a device for grinding the sampled bone matter, the device for grinding the bone matter being configured to receive the collecting tube (8) of the sample-taking device (1) and to grind the bone matter contained in said collecting tube (8).

7. Assembly according to claim 6, **characterized in that** the device for grinding the sampled bone matter is a portable device, for example a grinding gun.

8. Assembly according to either claim 6 or claim 7, **characterized in that** it further comprises a bead positioned within the collecting tube (8) containing the sampled bone matter.

9. Assembly according to any of claims 6 to 8, **characterized in that** the device for grinding bone matter is provided with an adapter (32) for tubes (8), said adapter (32) consisting of a removable receptacle configured to receive at least one collecting tube (8), preferably configured to receive two collecting tubes (8).

10. Mobile laboratory (40) formed from a transportable mobile container such as a rolling cart, for the collection, analysis and/or identification of biological materials, the mobile laboratory (40) comprising a sample-taking device (1) according to any of claims 1 to 3 or a sample-taking kit according to any of claims 4 to 5 or an assembly according to any of claims 6 to 9, and being configured to define an isolated workspace for a human operator.

11. Mobile laboratory (40) according to claim 10, **characterized in that** it further comprises a drill string comprising a rotary tool such as a portable power tool, an orthopedic drill, or even a dental motor; and a vice for holding bone fragments during drilling.

12. Mobile laboratory (40) according to either claim 10 or claim 11, **characterized in that** it further comprises a means of genetic analysis such as automatic DNA extraction.

13. Use of a device (1) according to any of claims 1 to 3 for the sampling of bone matter, **characterized in that** if the sample is taken with the device (1) directly on a human being, said human being is not living.
